# EUROPEAN PATENT SPECIFICATION

(11) **EP 1 454 635 B1**
(45) Date of publication and mention of the grant of the patent: **19.12.2007**
(21) Application number: 04005469.4
(22) Date of filing: 08.03.2004
(51) Int. Cl.: A61K 47/48, A61K 9/00

(54) **Tasteless, directly compressible, fast-dissolving complexes and pharmaceutical formulations thereof**
Geschmacklose, direkt verpressbare, schnelllösliche Komplexe und ihre pharmazeutischen Zusammensetzungen.
Complexes insipides, directement compressibles ,à dissolution rapide et leurs formulations pharmaceutiques

(30) Priority: 07.03.2003 US 383433
(43) Date of publication of application: 08.09.2004
(73) Proprietor: Ind-Swift Limited, Chandigarh - 160 002 (IN)
(72) Inventor: Wadhwa, Hardeep, Panchkula Haryana (IN)
(74) Representative: Kuhnen & Wacker

(56) References cited:
- WO-A-03/041683
- MAJID KHAN G ET AL: "Studies on drug release kinetics from ibuprofen-carbomer hydrophilic matrix tablets: influence of co-excipients on release rate of the drug" JOURNAL OF CONTROLLED RELEASE, ELSEVIER SCIENCE PUBLISHERS B.V., AMSTERDAM, NL, vol. 57, no. 2, 1 February 1999 (1999-02-01), pages 197-203, XP004157776 ISSN: 0168-3659

## Description

### Field of the Invention

The present invention relates to fexofenadine-carbomer complexes, pharmaceutical formulations thereof, and processes for producing the complexes and the formulations. More particularly, this invention relates to novel, tasteless, directly compressible, and fast-dissolving complexes of bitter salts of basic drugs and oral dosage forms thereof. The pharmaceutical formulations comprising the fexofenadine-carbomer complexes according to this invention can be used for treating symptoms associated with seasonal allergic rhinitis or with chronic idiopathic urticaria.

### Background of the Invention

Conventional oral solid dosage forms are difficult to administer. In particular children and elderly patients often experience difficulty in swallowing tablets and capsules. Moreover, it is inconvenient to comply with solid oral dosages during travels. Addressing these disadvantages, drugs have been formulated as chewable, dispersible or mouth-dissolving tablets, as dry powders for reconstitution, or as liquid oral dosage forms. These formulations allow greater exposure of the drug to the taste buds, resulting in problems of patient compliance when bitter or unpleasant tasting drugs are to be administered.

Consequently, various taste-masking techniques have been devised. The known arts address, in various preferred ways, the problems in formulating bitter tasting drugs. For drugs having minor bitterness, conventional taste masking techniques, such as the use of sweeteners and flavoring agents, are employed. However, for highly bitter drugs, techniques like complexation, fluidized bed coating, microencapsulation and solid dispersion involving the use of certain polymers are employed. These techniques are either very technology-intensive, requiring sophisticated equipment, or involving the use of organic solvents. The use of organic solvents generates regulatory and safety concerns. There are safety concerns particularly with respect to pediatric patients. Additionally, in many instances, a very large amount of polymer is used for taste masking or the polymer is such that the drug is released in the intestines. Further, the polymers used in taste masking adversely affect the release patterns of the drugs, and consequently the drugs' bioavailability.

The known arts fail to effectively address the above disadvantages. In particular, the known techniques have proved to be inefficient in masking bitter tasting salts of basic drugs. An example of such a bitter tasting salt of a basic drugs is fexofenadine hydrochloride. Fexofenadine hydrochloride is a histamine H₁-receptor antagonist with the chemical name (±)-4-[1-hydroxy-4-[4-(hydroxydiphenylmethyl)-1-piperidinyl]-butyl]-α,α-dimethylbenzene acetic acid hydrochloride. The molecular weight of this histamine H₁-receptor antagonist is 538.13, and its empirical formula is C₃₂H₃₉NO₄ HCl. Fexofenadine hydrochloride is indicated for the relief of symptoms associated with seasonal allergic rhinitis and with chronic idiopathic urticaria in adults and in children of and above the age of 6 years.

The recommended dose of fexofenadine hydrochloride is 30 mg twice daily for children between 6 and 11 years and 60 mg twice daily for adults and children above 11 years. The solid dosage forms available are either 60 mg capsules or 30/60 mg film coated tablets, which are to be swallowed orally, thus making the drug administration difficult, especially for children, elderly patients and during travel. Therefore it would be useful to devise a taste masked, granular, directly compressible, fast dissolving, stable complex of fexofenadine hydrochloride, which can be used in orally-disintegrating, mouth-dissolving, dispersible or chewable tablets.

Use of cation-exchange resins (such as polysulfonic acid and polycarboxylic acid polymers and their potassium salts) to adsorb amine drugs or their pharmaceutical acid salts for taste-masking and sustained release has been reported. These resins form insoluble adsorbates or resinates through weak ionic bonding with oppositely charged drugs. The adsorbates thus maintain a low solution concentration of drug in a suspension free of soluble counterions. They dissociate only at an acidic pH and hence remain in complexed form in the neutral pH of saliva, providing no or very little bitter taste in the mouth. After ingestion and exposure to ions in stomach, the resinate dissociates and drug is eluted to be absorbed. This technique, when used for fexofenadine hydrochloride, did not yield the desired results. A large quantity of resin (potassium salt of cross linked polyacrylic copolymer - polyacrilin potassium USNF 18) - three times that of the drug -- was consumed for taste-masking. Moreover, it suffered from the major disadvantage of very poor binding and compressibility properties. This made the process unviable for tablets.

The art disclosed in the United States Patent No. 4, 808, 411 to Lu, et al. describes a polymer-carrier system devised to reduce the bitterness of erythromycin and its 6-O-methyl derivative, clarithromycin, by absorption to Carbopol®. The macrolide-Carbopol complexes are prepared by dissolving or slurrying predetermined ratios of drug and polymer in water or hydroalcoholic mixtures. Taste protection is further improved by encapsulating the adsorbate particles with enteric polymer coatings, such as hydroxypropyl methylcellulose phthalate (hereinafter referred to as "HPMCP"). The mechanism of macrolide-Carbopol complex, like that of ion-exchange resins, involves ionic bonding of the amine macrolide to the high molecular weight polyacrylic acid, such as Carbopol 934 P, thereby removing the drug from the solution phase in an ion-free suspension.

CLAR⁺ + R-COO⁻ → R-COO⁻CLAR⁺

After ingestion, endogenous cations displace the drug from the polymer complex in the gastrointestinal tract to achieve bioavailability.

R-COO⁻CLAR⁺ + H⁺ or Na⁺ → CLAR⁺ + R-COOH or R-COO⁻Na⁺

Carbopol is advantageous over conventional ion-exchange resins in this application. It readily swells, allowing rapid cation exchange. Another advantage is that it dissolves in neutral buffers.

Presuming the technique disclosed in Lu et al. would be useful in the complexation and taste-masking of fexofenadine hydrochloride, it was employed, and surprisingly it was found that the bitterness remained the same. It was presumed that this was due to the incomplete reaction between fexofenadine salt and Carbomer due to the non-availability of a free amino group in the hydrochloride salt.

United States Patent No. 4,101,651 to Kobayashi, et al. discusses a process for granulation of a bitter drug, midecamycin, with a large amount of ethyl cellulose and volatile organic solvents.

United States Patent No. 4,916,161 to Patell, et al. discloses a process for wet granulating a mixture of ibuprofen and HPMCP with an aqueous composition in which the HPMCP is at least partly soluble to affect an improvement in the taste of ibuprofen. HPMCP provides an enteric effect, releasing the drug in the intestines.

United States Patent No. 5,188,839 to Pearmain, et al. discloses a taste-masked chewable tablet of cimetidine containing a copolymer of dimethyl ethyl methacrylate and neutral methacrylic acid ester as a granulating and binding agent, again involving the use of organic solvents.

PCT International Publication No. WO 00/76479 discloses a taste-masked composition comprising a bitter tasting drug with a combination of two enteric polymers, a methacryclic acid copolymer and a phthalate polymer, in an organic solvent and recovering the composition from the solution thereof.

United States Patent No. 4,865,851 to James, et al. discloses a lipid-based microencapsulation for taste-masking of cefuroxime axetil in particulate form coated with an integral coating of lipid or a mixture of lipids. It requires a highly sophisticated hot-melt granulation technique and may have an adverse effect on heat-sensitive molecules and on drug release.

PCT International Publication No. WO 2000009639 describes the formation of microspheres, which are then coated in a fluidized bed coater with a non-aqueous solution of certain polymers such as ethylcellulose and hydroxypropylcellulose.

United States Patent No. 6,027,746 to Lech, et. al. describes a chewable soft gelatin-encapsulated pharmaceutical adsorbate containing the drug absorbed onto the flakes of Mg trisilicate or SiO₂, which are then dispersed in a solid or liquid fill material containing flavorings, sweeteners, emulsifiers and the like.

The methods for taste masking the bitter salt of a basic drug disclosed in the prior art were found to be quite complicated, involving the use of sophisticated equipment or organic solvents. Some of them affect the release of the drug, while in others the taste-masked drug particles are coated or microencapsulated or are in the form of microspheres, which, if compressed to form tablets, may have their coating damaged and thus may give a bitter taste. Therefore, a need existed for a simple, aqueous method of taste-masking fexofenadine hydrochloride and other similar basic drugs and their pharmaceutical salts (whose free amino group has been reacted with an acid) so as to produce a taste-masked complex of the drug directly in granular form having a good compressibility, thus making it suitable for all types of tablets, capsules, dry syrups and liquid dosage forms. Also a need existed to avoid the use of organic solvents for safety purposes. Further, there was the need to get good dissolution and bioavailability of the drug and to use a very simple, environmentally friendly and cost-effective technique, avoiding sophisticated equipment and time-consuming processes. The use of a minimum amount of polymer was also desired.

### Summary of the Invention

In accordance with one embodiment, there is provided a fexofenadine-carbomer complex, which is a tasteless complex of a bitter acid salt of a basic drug with an acidic polymer. In this case the bitter salt of a basic drug is fexofenadine hydrochloride, and the acidic polymer is carbomer. The complex of the basic drug is a tasteless, granular, directly compressible, fast dissolving and stable fexofenadine-carbomer complex.

In accordance with another embodiment, there is provided a pharmaceutical formulation comprising the fexofenadine-carbomer complex. The formulation further comprises a diluent, a sweetening agent, a flavoring agent, a coloring agent, a disintegrating agent, a disintegration enhancer, a dissolution enhancer and/or a lubricant. The complex can be obtained by removing an acid salt of fexofenadine employing an alkali, reacting the fexofenadine with an acidic polymer such as a carbomer, to enable complexation, and isolating the complex, and drying and sieving the same.

Preferably along with the fexofenadine-carbomer complex, the pharmaceutical formulation further comprises mannitol as a preferred diluent, aspartame as a preferred sweetening agent, fruit flavor as a preferred flavoring agent, sunset yellow as a preferred coloring agent and microcrystalline cellulose as a preferred disintegrating agent, citric acid, sodium bicarbonate or crosscarmellose sodium as preferred disintegration and dissolution enhancers, and talcum or magnesium stearate, colloidal silicon dioxide, glyceryl behenate, or glyceryl palmitostearate as preferred lubricants.

The pharmaceutical formulation preferably is in a solid oral dosage form selected from tablets, capsules, pills, granules and dry syrups. The pharmaceutical formulation comprises a tasteless complex of a basic drug, fexofenadine, wherein the tasteless complex is a fexofenadine-carbomer complex. The formulation can be adapted for a twice daily dose regimen. The formulation preferably comprises a potency equivalent to 30 mg to 180 mg of fexofenadine hydrochloride. The formulation can comprise a potency equivalent to 30 mg of fexofenadine hydrochloride, which is desirably adapted for a twice daily dose regimen for children of 6 to 11 years, a potency equivalent to 60 mg of fexofenadine hydrochloride, which is desirably adapted for a twice daily dose regimen for children above 11 years and adults, a potency equivalent to 120 mg of fexofenadine hydrochloride, which is desirably adapted for a twice daily dose regimen for adults as the maximum dose, or a potency equivalent to 180 mg of fexofenadine hydrochloride, which is desirably adapted for a once daily dose regimen for children above 11 years and adults.

Preferably, a solid oral dosage form of a pharmaceutical formulation comprises a tasteless complex of a basic drug, fexofenadine, wherein the solid oral dosage form is a tablet. The tablet can be chewable, orally disintegrating, mouth dissolving and/or dispersible.

In accordance with still another embodiment, there is provided a process for producing a pharmaceutical formulation comprising a fexofenadine-carbomer complex which is a tasteless complex of a basic drug, fexofenadine. The process comprises sieving a dried tasteless, granular complex of a basic drug through suitable meshes in order to get a desired particle size, mixing it with one or more components selected from diluents, disintegrating agents, coloring agents, disintegration and dissolution enhancers, sweetening agents, flavoring agents and lubricants thoroughly for a predetermined period of time under controlled temperature and humidity conditions to form an oral solid dosage formulation. In one embodiment, the tasteless complex of the basic drug is a fexofenadine-carbomer complex, the diluent is mannitol, the sweetening agent is aspartame, the disintegrating agent is microcrystalline cellulose , the disintegration and dissolution enhancers are selected from citric acid, sodium bicarbonate and crosscarmellose sodium , the coloring agent is sunset yellow lake, the flavoring agent is fruit flavor and the lubricant is talcum, magnesium stearate, colloidal silicon dioxide, glyceryl behenate, glyceryl palmitostearate or a mixture thereof. A mixture of these ingredients with a tasteless, granular, directly compressible fexofenadine-carbomer complex may also be compressed to form a dispersible or mouth-dissolving or orally-disintegrating or chewable tablet.

In accordance with yet another embodiment, there is provided a process for producing tasteless complex of a basic drug by removing an acidic salt of the basic drug employing an alkali, reacting the basic drug with an acidic polymer to enable complexation, and isolating, drying and sieving the complex to produce a tasteless, granular, directly compressible, fast-dissolving and stable complex of the basic drug. Preferably, the basic drug is fexofenadine, the acidic salt of the basic drug is the hydrochloride salt of fexofenadine, the basic drug is reacted with the acidic polymer in an aqueous medium, and the alkali employed is a hydroxide of an alkali metal, such as NaOH or KOH, used in an equimolar quantity of the salt of the basic drug. Preferably, the acidic polymer used is a carbomer and the drug-polymer ratio ranges from 1:0.25 to 1:1. More preferably, the drug-polymer ratio ranges from 1:0.5 to 1:0.6. Preferably, the complex is isolated by centrifugation and filtration and is dried at 40°-45°C.

A process for characterizing a tasteless complex of a basic drug can be realized by analyzing at least one property of the isolated complex of the basic drug. Preferably, the basic drug is fexofenadine, and the acidic salt of the basic drug is the hydrochloride salt of fexofenadine. The analyzing at least one property of the isolated complex of the basic drug comprises at least one analysis selected from the group consisting of pH-solubility profiling, assessing its melting point, assessing its water content, analyzing the complex by high performance liquid chromatography (HPLC), and analyzing the complex by differential scanning calorimetry (DSC). Preferably, the tasteless, granular, directly compressible complex of the basic drug thus isolated and characterized is a fexofenadine-carbomer complex.

Preferably, in the process of the invention a tasteless, directly compressible, granular, stable and fast-dissolving complex of fexofenadine can be produced. The process comprises dispersing an equimolar quantity of fexofenadine hydrochloride in an aqueous solution of sodium hydroxide, stirring the composition sufficiently, thus changing the nature of the drug dispersion, adding slowly carbomer in the form of an aqueous gel while stirring in an amount such that the drug:polymer concentration ratio ranges from 1:0.5 to 1:0.6, enabling thickening of the dispersion of drug upon gradual addition of carbomer gel until complete complexation takes place; enabling separation of two phases, one containing the tasteless complex of the basic drug, fexofenadine, and carbomer, and the other containing water. Preferably, the solid phase is separated by centrifugation and filtration and dried at 40-45°C until a moisture content of 2-5% is achieved. The tasteless, directly compressible and granular complex of fexofenadine thus obtained is forced through suitable meshes to get a desired particle size for use in dispersible, chewable, mouth-dissolving and/or orally disintegrating tablets. Preferably, the stage of complexation is reached and phase separation starts with the addition of carbomer in an amount slightly less than 50% of drug and continues until 60%. Further quantities of carbomer greater than 60% do not make much difference in taste, but instead makes the two phases miscible with each other, making the separation, filtration and drying of the complex all the more difficult. The quantity of sodium hydroxide and the quantity of carbomer used affect the formation of a tasteless, easily separable, filterable, stable, granular, directly compressible and fast-dissolving complex of fexofenadine.

### Description of the Drawings

These and other features and advantages of the present invention will be better understood by reference to the following detailed description when considered in conjunction with the accompanying drawings wherein:
FIG. 1(a) is a graph showing the comparative dissolution profile of orally disintegrating uncoated fexofenadine tablets according to the invention at pH 1.2 relative to that of a conventional film coated table of fexofenadine HCl.
FIG. 1(b) is a graph showing the comparative dissolution profile of dispersible uncoated fexofenadine tablets according to the invention at pH 1.2 relative to that of a conventional film coated table of fexofenadine HCl.
FIG. 1(c) is a graph showing the comparative dissolution profile of mouth dissolving uncoated fexofenadine tablets according to the invention at pH 1.2 relative to that of a conventional film coated table of fexofenadine HCl.
FIG. 2(a) is a graph showing the comparative dissolution profile of orally disintegrating uncoated fexofenadine tablets according to the invention at pH 6.8 relative to that of a conventional film coated table of fexofenadine HCl.
FIG. 2(b) is a graph showing the comparative dissolution profile of dispersible uncoated fexofenadine tablets according to the invention at pH 6.8 relative to that of a conventional film coated table of fexofenadine HCl.
FIG. 2(c) is a graph showing the comparative dissolution profile of mouth dissolving uncoated fexofenadine tablets according to the invention at pH 6.8 relative to that of a conventional film coated table of fexofenadine HCl.
FIG. 3 is a graph showing the plasma concentration-time curve after a single dose administration of a tasteless, orally disintegrating, uncoated fexofenadine tablet according to the invention having a potency equivalent to 120 mg of fexofenadine hydrochloride.

### Detailed Description

Fexofenadine, an H₁-histamine antagonist drug, is used in the form of the pharmaceutically acceptable hydrochloride salt (MW-538), and its structure is as follows:

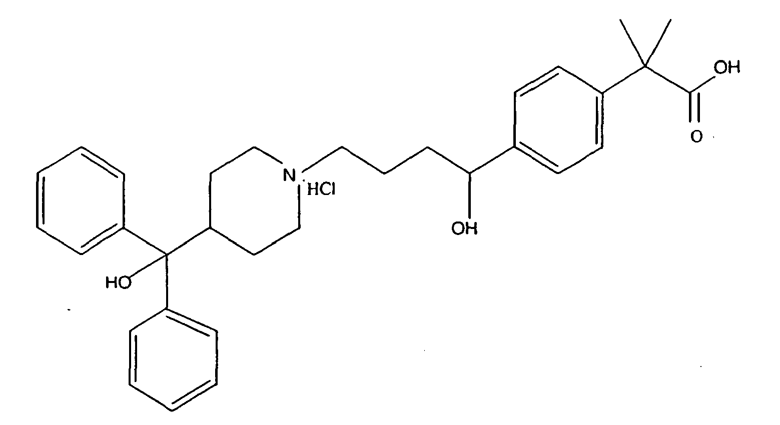

As can be seen from the structure, the nitrogen atom present in the ring is being utilized by the HCl moiety, leaving nothing for the carboxyl group of polyacrylic acid or carbomer to react with. Hence, when an aqueous dispersion of fexofenadine hydrochloride is mixed with carbomer gel as such, even in a ratio of 1:1, nothing happens, and no taste masking occurs. It was discovered to free only the nitrogen atom of fexofenadine hydrochloride by first adding just a sufficient amount of sodium hydroxide, and then adding carbomer gel.

As used herein, the term "carbomer" refers to a polymer of acrylic acid crosslinked with allyl ethers of sugars, such as sucrose, or polyalcohols, such as penta erythritol. Such carbomers are typically high molecular weight polymers. Preferably the carbomers contain not less than 56% and not more than 68% of carboxylic acid (-COOH) groups. Exemplary carbomers useful in the invention are commercially available from B.F. Goodrich under the name Carbopol®.

The amount of sodium hydroxide is preferably carefuly controlled. If less sodium hydroxide is used than what is required to free only the nitrogen of fexofenadine, then the complete reaction does not take place between fexofenadine and carbomer and proper taste masking does not occur. On the other hand, if excess sodium hydroxide is used, it makes the carbomer swell and its gel very thick, thus not allowing proper stirring and separation of the tasteless complex of fexofenadine to occur. Further, while the taste of the complex thus formed is not bitter, when kept in the mouth, it has a slimy and sticky feel and does not disintegrate or dissolve quickly. Both when there is insufficient sodium hydroxide and when there is excess sodium hydroxide, the tasteless fexofenadine complex formation does not properly occur and does not occur at all if no sodium hydroxide or other alkali is added. Only if an equimolar quantity of sodium hydroxide or other alkali solution is first added to fexofenadine hydrochloride and then, after its nitrogen group is free, carbomer aqueous gel is added in a drug:polymer ratio ranging from 1:0.5 to 1:0.6, then a complete phase separation results and an easily filterable, stable, granular, directly compressible, tasteless and fast-dissolving fexofenadine complex is formed with the properties described in Table-I.

### Process for Preparation of Fexofenadine-Carbomer Complex

Fexofenadine hydrochloride was dispersed in an aqueous solution of sodium hydroxide, the latter in an equimolar quantity. After sufficient stirring, which changed the nature of the drug dispersion, a carbomer was added slowly in the form of an aqueous gel with simultaneous proper stirring. The carbomer was added in a concentration sufficient to provide a drug:polymer ratio ranging from 1:0.5 to 1:0.6. The dispersion of drug thickened more and more with the gradual addition of carbomer gel until a stage was reached where complete complexation took place and separation of two phases occured, one phase containing the tasteless complex of the basic drug-fexofenadine and a carbomer, and the other one containing water. The solid phase was separated by centrifugation and filtration and dried at 40-45°C until a moisture content of 2-5% was achieved. The tasteless, directly compressible and granular complex of fexofenadine thus obtained was forced through suitable meshes to get a desired particle size for use in dispersible, chewable, mouth dissolving and/or orally disintegrating tablets described further. The stage of complexation is reached and phase separation starts when the amount of carbomer added is slightly less than 50% of the amount of drug present and continues until the amount of carbomer is about 60% of the amount of drug. Further quantities of carbomer more than 60% of the amount of drug do not make much difference in taste; rather additional carbomer makes the two phases miscible with each other, making the separation, filtration and drying of complex all the more difficult. Thus, both the quantity of sodium hydroxide or other alkali and the quantity of carbomer used are very important for the formation of a tasteless, easily separable, filterable, stable, granular, directly compressible and fast-dissolving complex of fexofenadine.

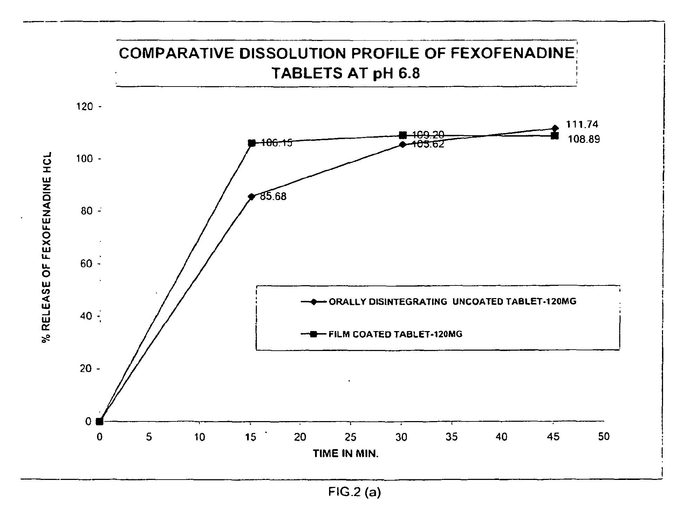

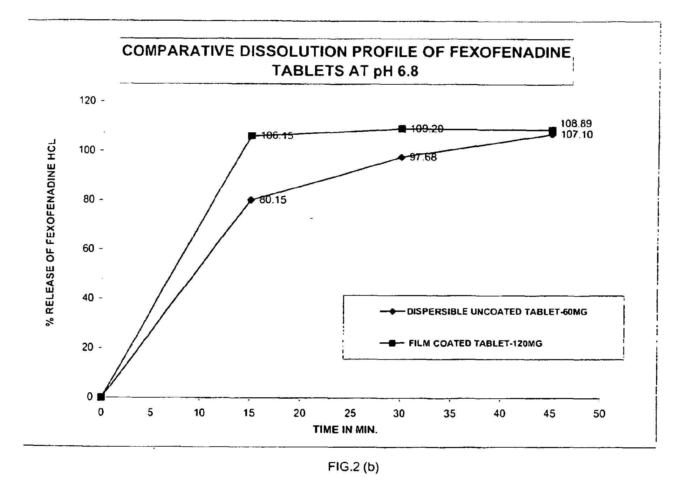

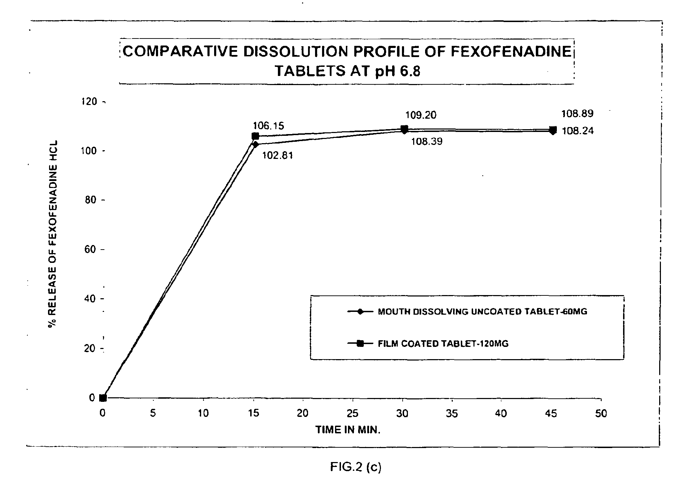

The complex of fexofenadine with carbomer is better than fexofenadine hydrochloride in many respects. In addition to its bitterless taste, it has a pH-independent solubility up to pH 6.0, and then at pH 7.0 the solubility increases and becomes equal or more than that of fexofenadine hydrochloride. The latter has significantly less solubility at pH 1.2 and at pH 5.0 (less than that of the complex) and high solubility at pH 3.0 & 4.0, making its absorption in the gastrointestinal tract pH-dependent. This is not the case with the fexofenadine-carbomer complex, which has almost the same solubility at all pH levels from 1.2 to 6.0, ranging from 0.4 to 0.6 mg/ml as shown in Table-I. Even the rate of dissolution of an orally disintegrating, dispersible and mouth-dissolving tablet made from the above complex at pH 1.2 is much higher than that of a conventional film coated tablet of fexofenadine hydrochloride as shown in Figs. I(a), I(b) & I(c). At pH 6.8, all are equivalent from 15 minutes onwards as shown in Figs. 2(a), 2(b) & 2(c). The reason for the better dissolution profile at pH 1.2 may be the quick dissociation of the fexofenadine-carbomer complex at an acidic pH and subsequent solubility of the fexofenadine base in acid. At pH 6.8, the carbomer dissolves readily, and the drug is released again. This means that the onset of action of the oral dosage form made out of the fexofenadine-carbomer complex will be faster than the tablet of fexofenadine hydrochloride, and more so if it is a dispersible or chewable or orally disintegrating or mouth dissolving tablet, while not compromising with the percentage of drug available to the body. Moreover, in addition to the advantages of a much better taste, the fexofenadine-carbomer complex is obtained directly in granular and directly compressible form after it is separated, dried and passed through suitable meshes. There is no need for further granulation using binders, as is required for fexofenadine hydrochloride film coated tablets.

The dispersible or orally disintegrating or mouth-dissolving tablet of fexofenadine prepared from the above tasteless, granular, directly compressible, stable and fast-dissolving complex has further advantages of ease of administration with or without water, ease of providing accurate and divisible doses and patient compliance. The stability studies carried out with the above pure complex as well as with its dispersible, orally disintegrating and mouth dissolving tablet using different flavors and sweetening agents do not show any significant degradation of the drug at any of the stability conditions with all impurities well within the limits. Even the physical properties and dissolution profiles are least affected during the shelf life.

### Process for Preparation of an Orally Disintegrating/Mouth Dissolving Tablet of Fexofenadine:

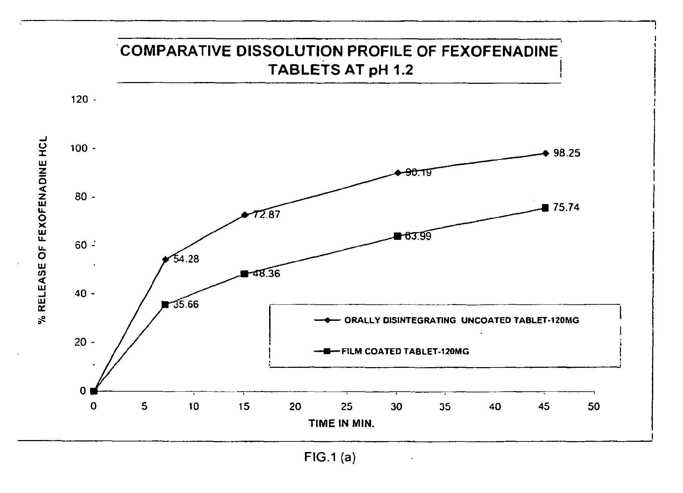

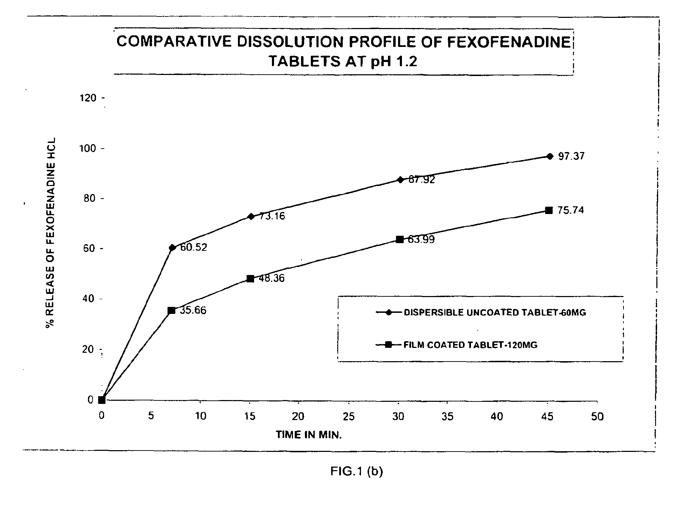

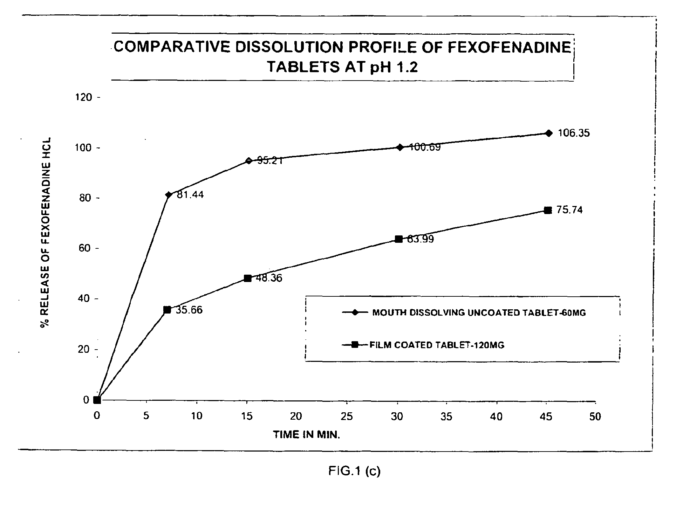

A fexofenadine-carbomer complex prepared, isolated and characterized as described above was obtained directly in a tasteless, granular, directly compressible, fast-dissolving and stable form, so it was used as such with a desired particle size for the preparation of the above-mentioned tablet. A quantity of the complex equivalent to the desired dose of fexofenadine hydrochloride (30mg/60mg/120mg) was taken (15-40% by weight of composition) and dry mixed with the standard pharmaceutical excipients used for a mouth dissolving/orally disintegrating tablet, e.g., diluents like directly compressible mannitol (30 - 60% by weight of composition); sweetening agent like aspartame; flavoring agents; coloring agents; disintegrating agents like microcrystalline cellulose (5-20% by weight of tablet); disintegration and dissolution enhancers like citric acid, sodium bicarbonate and crosscarmellose sodium; and lubricants like talcum, magnesium stearate, colloidal silicon dioxide, glyceryl behenate, and glyceryl palmitostearate. The above excipients and the drug were geometrically dry mixed and compressed at a slightly lower compression pressure under controlled temperature and humidity conditions. The tablet thus formed disintegrated within 30-60 seconds in the mouth without any water, giving a pleasant mouth feel and leaving no residue in the oral cavity after the saliva containing the dispersed or dissolved drug and excipients was swallowed. Fast mouth dissolving/oral disintegrating action accompanied by a fast dissolution profile of this tablet at pH 1.2 as compared to a conventional film coated tablet of fexofenadine hydrochloride [Figures 1(c) & 1(a)] (more than 85% dissolving in 30 minutes) showed that it should lead to faster absorption and onset of action. It has the further advantages of patient convenience (no need of water), patient compliance, accurate and divisible doses.

### Process for Preparation of a Dispersible Tablet of Fexofenadine:

The tasteless, granular and directly compressible fexofenadine-carbomer complex of a desired particle size was used in a quantity equivalent to the desired dose of fexofenadine hydrochloride (30mg/60mg/120mg), 15-40% by weight of the composition, and dry mixed geometrically with the standard pharmaceutical excipients used for a dispersible tablet, for example, a diluent and disintegrating agent like directly compressible microcrystalline cellulose (30-60% by weight of tablet); a sweetening agent like aspartame; flavoring agents; a coloring agent; disintegration enhancers like crosscarmellose sodium; and lubricants like talcum and magnesium stearate. The mixture was compressed like an ordinary tablet. The tablet thus formed dispersed in 30-60 seconds in 10-15ml water at 25 °C, which when swallowed gave a pleasant taste and left no residue in the oral cavity. Fast disintegrating action accompanied by a fast dissolution profile of this tablet at pH 1.2 as compared to a conventional film coated tablet of fexofenadine hydrochloride [Fig. 1(b)] (more than 80% dissolving in 30 minutes) showed that it should lead to faster absorption and onset of action. It has the further advantages of patient convenience, patient compliance, and accurate and divisible doses.

### Pharmacokinetic studies

A single dose, pharmacokinetic study of a fexofenadine orally disintegrating uncoated tablet containing fexofenadine-carbomer complex equivalent to 120mg of fexofenadine hydrochloride prepared as described above was carried out in healthy adult male human volunteers under fasting conditions. The orally disintegrating tablet was not swallowed but was allowed to dissolve/disperse in the mouth during administration of the drug to the volunteers.

Plasma samples collected at different time intervals up to 48 hrs. were assayed for fexofenadine using a validated high-performance liquid chromatographic procedure using a fluorescence detector described in "Determination of Terfenadine and Terfenadine acid metabolite in plasma using solid phase extraction and HPLC with fluorescence detection" J. Chromatogr 1991 (570), p. 139-148.

### Example-1

### Preparation of Fexofenadine Orally Disintegrating/Mouth Dissolving Tablet:

A preferred composition according to the invention is as follows:

| **INGREDIENTS** | **QTY. (MG/TAB.)** | **QTY. (MG/TAB.)** | **QTY. (MG/TAB.)** |
|---|---|---|---|
| | **FOR 30MG** | **FOR 60MG** | **FOR 120MG** |
| | **(1)** | **(2)** | **(3)** |
| Fexofenadine-carbomer Complex (having a potency of about 60%) equivalent to 30/ 60/120mg of Fexofenadine HCl -Directly compressible | 50 | 100 | 200 |
| Mannitol - Directly Compressible | 157 | 289 | 213 |
| Microcrystalline Cellulose - Directly Compressible | 35 | 70 | 70 |
| Crosscarmellose Sodium | 9 | 18 | 18 |
| Aspartame | 12 | 24 | 24 |
| Flavor - Mixed Fruit | 20 | 40 | 40 |
| Talcum | 3 | 6 | 6 |
| Magnesium Stearate | 3 | 6 | 6 |
| Color - Sunset Yellow Lake | 3 | 6 | 6 |
| Citric Acid | 6 | 12 | 12 |
| Sodium Bicarbonate | 2.5 | 5 | 5 |
| Aerosil (Colloidal silicon dioxide) | -- | 6 | -- |
| Glyceryl Behenate/ Palmitostearate | -- | 18 | -- |
| | 300.5mg | 600mg | 600mg |

### Process for Preparation of a Fexofenadine Orally Disintegrating/Mouth Dissolving Tablet:

A fexofenadine-carbomer complex was passed through suitable meshes. It was mixed with mannitol and microcrystalline cellulose one by one after sifting each diluent through a 44# mesh. Sunset yellow lake was passed through an 85# mesh and geometrically mixed with the above portion. Then citric acid and sodium bicarbonate were added one by one to the above mixture after passing each through a 60# mesh and protecting from moisture. Lubricants (talc, magnesium stearate, colloidal silicon dioxide, glyceryl behenate, and glyceryl palmitostearate) were mixed separately with aspartame, crosscarmellose sodium and then flavor after sifting each of them through a 60# mesh. This was then geometrically added to the above mixture, mixed thoroughly for 20-30 minutes and compressed at a pressure slightly less than that used for conventional tablets and under controlled temperature and humidity conditions. They were then strip-packed after complete testing.

### Example-2

### Preparation of a Dispersible Tablet of Fexofenadine:

| **INGREDIENTS** | **QUANTITY (MG/TABLET)** | **QUANTITY (MG/TABLET)** |
|---|---|---|
| | **FOR 60MG STRENGTH** | **FOR 120MG STRENGTH** |
| Fexofenadine-Carbomer Complex | 100 | 200 |
| (having a potency of about 60%) | | |
| equivalent to 60/120mg of Fexofenadine | | |
| HCl- Directly Compressible Microcrystalline Cellulose - | 157 | 314 |
| Directly Compressible Aspartame | 10 | 20 |
| Crosscarmellose Sodium | 9 | 18 |
| Talcum | 3 | 6 |
| Magnesium Stearate | 3 | 6 |
| Flavor - Mixed Fruit | 15 | 30 |
| Color - Sunset Yellow Lake | 3 | 6 |
| | 300mg | 600mg |

### Process for Preparation of a Dispersible Tablet of Fexofenadine:

A fexofenadine-carbomer complex was passed through 44 BSS No. mesh and retained on a 60# mesh. It was then mixed with directly compressible microcrystalline cellulose, sifted through a 44# mesh. Color was sifted through a fine mesh and geometrically mixed with the above. Lubricants (talcum and magnesium stearate) were mixed with aspartame, crosscarmellose sodium and flavor one by one after sifting each through a 60# mesh. This was then geometrically mixed with the above mixture for 20-30 minutes and then compressed like ordinary tablets, which were then strip-packed after complete testing.

### Example-3

### Pharmacokinetic profile

The pharmacokinetic study of a single dose of an orally disintegrating uncoated fexofenadine tablet containing a fexofenadine-carbomer complex equivalent to 120mg of fexofenadine hydrochloride was carried out in healthy adult male human subjects under fasting conditions. The human volunteers were selected from males ranging in age from 18-45 years. They were medically fit persons with a normal weight to height ratio, with no history of allergy to the drug or any infectious disease or any liver, kidney or cardiovascular disorder or alcoholism or drug dependence. They were screened after a complete physical, biochemical and haematological examination. Each subject received a single dose of 120mg equivalent of fexofenadine hydrochloride after overnight fasting. The orally disintegrating tablet was kept in the mouth without water so as to allow it to disperse or dissolve, and the saliva was then swallowed. No food was allowed for 2 hours after administering the drug.

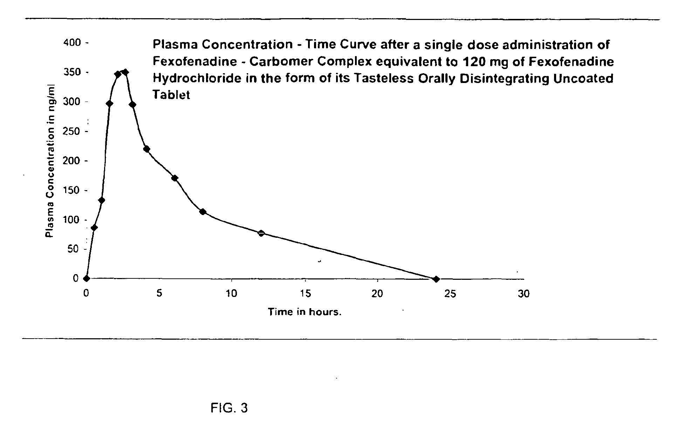

A standard diet was provided to the volunteers during the study. Blood samples were collected prior to dosing (0 hr.) and at 0.5, 1.0, 1.5, 2.0, 2.5, 3.0, 4.0, 6.0, 8.0, 12.0, 24.0 and 48.0 hrs. after dosing. The collected blood samples were transferred to heparinised collection tubes and centrifuged to be stored at - 20°C till the time of analysis. Plasma samples were analysed using HPLC and a fluorescence detector, the results of which showed the plasma concentration - time curve for fexofenadine hydrochloride as shown in Fig. 3. The pharmacokinetic parameters are shown in Table II.

**TABLE-I**

| **COMPARATIVE DATA OF FEXOFENADINE HYDROCHLORIDE & FEXOFENADINE-CARBOMER COMPLEX** | | | | | |
|---|---|---|---|---|---|
| **S. No.** | **Parameters** | **Fexofenadine Hydrochloride** | **Fexofenadine-Carbomer Complex** | | |
| 01. | Taste | Very bitter | Almost bitterless | | |
| 02. | Melting Point | 198°C- 208°C | 240°C - 260°C | | |
| 03. | PH | 2.25 - 2.75 | 4.00-5.00 | | |
| 04. | Water Content / Loss on Drying (LOD) | NMT 1 % (LOD) | 3 - 6% | | |
| 05. | Assay by HPLC | 99.0% | 54-66% | | |
| | (in terms of Fexofenadine HCl) | (on as such basis) | (on as such basis) | | |
| 06. | Differential Scanning Calorimetery | 202.40°C | 247.35°C | | |
| | (using Al crucible, open pan, 40 µl, temperature range of 25°C to 350°C @ 10°C/min under N2 atmosphere of 80 ml/min.) | (198.43°C-207.66°C) | (238.32°C- 260.76°C) | | |
| | | | Peak of Fexofenadine HCl disappears. | | |
| 07. | PH-solubility profile | | | | |
| | (mg of Fexofenadine HCl/ml) | | | | |
| | PH 1.2 (KCl Buffer) | 0.281 | 0.663 | | |
| | PH 3.0 (KCl Buffer) | 0.858 | 0.456 | | |
| | PH 4.0 (KCl Buffer) | 0.846 | 0.454 | | |
| | PH 5.0 (KH₂PO₄ Buffer) | 0.266 | 0.403 | | |
| | PH 6.0 (KH₂PO₄ Buffer) | 0.724 | 0.503 | | |
| | PH 7.0 (KH₂PO₄ Buffer) | 0.732 | 1.042 | | |
| 08. | Dissolution Profile at pH 1.2 | Conventional Fexofenadine HCl 120mg film coated tablet | Present Invention | | |
| | | | Orally disintegrating Uncoated Tablet-120mg | Dispersible Uncoated Tablet-60mg | Mouth dissolving Tablet-60mg |
| | | 35.66% | | | |
| | 7 min. | 48.36% | 54.28% | 60.52% | 81.44% |
| | 15 min. | 63.99% | 72.87% | 73.16% | 95.21% |
| | 30 min. | 75.74% | 90.19% | 87.92% | 100.69% |
| | 45 min. | | 98.25% | 97.37% | 106.35% |
| 09. | Dissolution profile at pH 6.8 | | | | |
| | 15 min. | 106.15% | 85.68% | 80.15% | 102.81% |
| | | | | | |
| | 30 min. | 109.20% | 105.62% | 97.68% | 108.39% |
| | | | | | |
| | 45 min. | 108.89% | 111.74% | 107.10% | 108.24% |
| 10. | Stability | Stable | Stable | | |

**TABLE-II**

| **PHARMACOKINETIC PARAMETERS AFTER A SINGLE DOSE ADMINISTRATION OF FEXOFENADINE-CARBOMER COMPLEX EQUIVALENT TO 120 MG OF FEXOFENADINE HCL IN THE FORM OF ITS TASTELESS ,ORALLY DISINTEGRATING, UNCOATED TABLET** | |
|---|---|
| **Parameter** | **Results** |
| Cₘₐₓ (ng/ml) | 413.3058 |
| Tₘₐₓ (hours) | 2.0833 |
| AUCₒ₋₁ (ng.h/ml) | 2005.5292 |
| AUC_{o-∞} (ng.h/ml) | 2727.53 |

While this invention has been described in detail with reference to certain preferred embodiments, it should be appreciated that the present invention is not limited to those precise embodiments. Rather, in view of the present disclosure, which describes the current best mode for practicing the invention, many modifications and variations would present themselves to those skilled in the art without departing from the scope and spirit of this invention.

## Claims

1. A fexofenadine-carbomer complex comprising fexofenadine complexed with a carbomer.

2. The fexofenadine-carbomer complex according to claim 1, wherein the ratio of fexofenadine to carbomer in the complex ranges from 1:0.25 to 1:1.

3. The fexofenadine-carbomer complex according to claim 1, wherein the ratio of fexofenadine to carbomer in the complex ranges from 1:0.5 to 1:0.6.

4. The fexofenadine-carbomer complex according to claim 1, wherein the complex is tasteless.

5. The fexofenadine-carbomer complex according to claim 4, wherein the complex is directly compressible and fast dissolving

6. The fexofenadine-carbomer complex according to claim 1, wherein the complex is obtained by:
contacting a fexofenadine acid salt with an alkali; and
reacting the fexofenadine with a carbomer under conditions sufficient to enable complexation.

7. The fexofenadine-carbomercomplex according to claim 6, wherein the contacting and reacting steps are performed in an aqueous medium.

8. A process for producing a complex of a basic drug comprising:
removing an acid salt of the basic drug employing an alkali; and
reacting the basic drug with an acidic polymer under conditions sufficient to form a complex of the basic drug with the acidic polymer.

9. The process according to claim 8, wherein the removing and reacting steps are performed in an aqueous medium.

10. The process according to claim 8, further comprising isolating, drying and sieving the complex.

11. The process according to claim 10, wherein the complex is isolated by centrifugation and filtration.

12. The process according to claim 10, wherein the isolated complex is dried at 40°-45°C.

13. The process according to claim 10, further comprising characterizing the complex.

14. The process according to claim 13, wherein the complex is **characterized by** analyzing at least one of property of the complex.

15. The process according to claim 13, wherein the complex is **characterized by** assessing its pH-solubility profile, assessing its melting point, assessing its water content, analyzing it by high performance liquid chromatography (HPLC) and/or analyzing it by differential scanning calorimetry (DSC).

16. The process according to claim 8, wherein the basic drug is fexofenadine.

17. The process according to claim 8, wherein the acidic salt of the basic drug is a hydrochloride salt of fexofenadine.

18. The process according to claim 8, wherein the complex is a fexofenadine-carbomer complex.

19. The process according to claim 8, wherein the alkali employed is a hydroxide of an alkali metal.

20. The process according to claim 8, wherein the alkali is NaOH or KOH.

21. The process according to claim 8, wherein the alkali and the salt of the basic drug are provided in equi-molar quantities.

22. The process according to claim 8, wherein the acidic polymer is a carbomer.

23. The process according to claim 8, wherein the ratio of the basic drug to the acidic polymer ranges from 1:0.25 to 1:1.

24. The process according to claim 8, wherein the ratio of the basic drug to the acidic polymer ranges from 1:0.5 to 1:0.6.

25. The process according to claim 8, further comprising obtaining the complex in a tasteless, granular, directly compressible, fast dissolving and stable form.

26. The process according to claim 8, comprising
removing an acid salt of fexofenadine employing an alkali in an aqueous medium, wherein the alkali and the acid salt of fexofenadine are provided in equimolar quantities; and
reacting the fexofenadine with a carbomer in an aqueous medium under conditions sufficient to form a fexofenadine-carbomer complex.

27. A pharmaceutical formulation comprising a fexofenadine-carbomer complex and a pharmaceutically acceptable excipient.

28. The pharmaceutical formulation according to claim 27, further comprising at least one agent selected from sweetening agents and flavoring agents.

29. The pharmaceutical formulation according to claim 27, further comprising a sweetening agent selected from the group consisting of saccharides, aspartame, neotame and stevioside.

30. The pharmaceutical formulation according to claim 27, further comprising a flavoring agent.

31. The pharmaceutical formulation according to claim 27, further comprising at least one of a disintegrating agent and a disintegration/dissolution enhancer.

32. A pharmaceutical formulation according to claim 27, further comprising at least one disintegrating agent selected from the group consisting of starch derivatives, celluloses, and cellulose derivatives.

33. The pharmaceutical formulation according to claim 27, further comprising a dissolution/disintegration enhancer.

34. The pharmaceutical formulation according to claim 27, further comprising at least one dissolution/disintegration enhancer selected from the group consisting of citric acid, sodium bicarbonate and crosscarmellose sodium.

35. The pharmaceutical formulation according to claim 27, further comprising a coloring agent.

36. The pharmaceutical formulation according to claim 27, further comprising a diluent selected from the group consisting of mannitol, starches, lactose, sucrose, sorbitol, celluloses and mixtures thereof.

37. The pharmaceutical formulation according to claim 27, further comprising a lubricant.

38. The pharmaceutical formulation according to claim 37, wherein the lubricant is selected from the group consisting of talcum, magnesium stearate, calcium stearate, polyethylene glycols, colloidal silicon dioxide, fatty acids, glyceryl esters of fatty acids and mixtures thereof.

39. The pharmaceutical formulation according to claim 27, wherein the fexofenadine-carbomer complex is present in an amount ranging from 15 to 40% by weight based on the total weight of the formulation.

40. The pharmaceutical formulation according to claim 39, further comprising a diluent that is present in an amount ranging from 30 to 60% by weight based on the total weight of the formulation.

41. The pharmaceutical formulation according to claim 27, wherein the fexofenadine-carbomer complex is tasteless, directly compressible and fast-dissolving.

42. The pharmaceutical formulation according to claim 27, wherein the fexofenadine-carbomer complex is obtained by:
contacting a fexofenadine acid salt with an alkali; and
reacting the fexofenadine with a carbomer under conditions sufficient to enable complexation.

43. The pharmaceutical formulation according to claim 42, wherein the contacting and reacting steps are performed in an aqueous medium.

44. The pharmaceutical formulation according to claim 27, wherein the formulation is in a solid oral dosage form.

45. The pharmaceutical formulation according to claim 44, wherein the solid oral dosage form is selected from the group consisting of tablets, capsules, pills, granules and dry syrups.

46. The pharmaceutical formulation according to claim 27, wherein the formulation comprises a potency equivalent of fexofenadine hydrochloride ranging from 30 mg to 180 mg.

47. The pharmaceutical formulation according to claim 27, wherein the formulation comprises a potency equivalent to 30 mg of fexofenadine hydrochloride.

48. The pharmaceutical formulation according to claim 27, wherein the formulation comprises a potency equivalent to 60 mg of fexofenadine hydrochloride.

49. The pharmaceutical formulation according to claim 27, wherein the formulation comprises a potency equivalent to 120 mg of fexofenadine hydrochloride.

50. The pharmaceutical formulation according to claim 27, wherein the formulation comprises a potency equivalent to 180 mg of fexofenadine hydrochloride.

51. The pharmaceutical formulation according to claim 27, wherein the formulation is in the form of a tablet that is chewable, dispersible, orally disintegrating and/or mouth dissolving.

52. The pharmaceutical formulation according to claim 27, further comprising a diluent, a sweetening agent, a flavoring agent, a coloring agent, a disintegrating agent, a disintegration enhancer, a dissolution enhancer, and/or a lubricant.

53. A process for producing the pharmaceutical formulation comprising a fexofenadine-carbomer complex and a pharmaceutically acceptable excipient, the process comprising sieving the fexofenadine-carbomer complex in dried form through one or more meshes to obtain the fexofenadine-carbomer complex in a granular form of a desired particle size and mixing the sieved fexofenadine-carbomer complex with the excipient for a predetermined period of time under controlled temperature and humidity conditions to form an oral solid dosage formulation.

54. A process according to claim 53, further comprising sifting the excipient before mixing the sieved fexofenadine-carbomer complex with the excipient.

55. A process according to claim 53, further comprising compressing the mixture of the fexofenadine-carbomer complex and the excipient to produce the oral solid dosage formulation in the form of tablets.

## Patentansprüche

1. Fexofenadin-Carbomer-Komplex, mit einem Carbomer komplexiertes Fexofenadin umfassend.

2. Fexofenadin-Carbomer-Komplex nach Anspruch 1, wobei das Verhältnis des Fexofenadins zu dem Carbomer in dem Komplex in einem Bereich von 1:0,25 bis 1:1 liegt.

3. Fexofenadin-Carbomer-Komplex nach Anspruch 1, wobei das Verhältnis des Fexofenadins zu dem Carbomer in dem Komplex in einem Bereich von 1:0,5 bis 1:0,6 liegt.

4. Fexofenadin-Carbomer-Komplex nach Anspruch 1, wobei der Komplex geschmacksneutral ist.

5. Fexofenadin-Carbomer-Komplex nach Anspruch 4, wobei der Komplex direkt pressbar und schnell löslich ist.

6. Fexofenadin-Carbomer-Komplex nach Anspruch 1, wobei der Komplex erhalten wird durch:
in Kontakt bringen eines Fexofenadin-Säuresalzes mit einer Lauge; und
Umsetzen des Fexofenadins mit einem Carbomer unter Bedingungen, die ausreichend sind eine Komplexierung zu ermöglichen.

7. Fexofenadin-Carbomer-Komplex nach Anspruch 6, wobei die Schritte des in Kontakt bringens und des Umsetzens in einem wässrigen Medium durchgeführt werden.

8. Verfahren zum Herstellen eines Komplexes eines basischen Arzneimittels umfassend:
Entfernen eines Säuresalzes von einem basischen Arzneimittel unter Verwenden einer Lauge; und
Umsetzen des basischen Arzneimittels mit einem sauren Polymer unter Bedingungen, die ausreichen einen Komplex des basischen Arzneimittels mit dem sauren Polymer zu bilden.

9. Verfahren nach Anspruch 8, wobei die Schritte des Entfernens und des Umsetzens in einem wässrigen Medium durchgeführt werden.

10. Verfahren nach Anspruch 8, ferner das Isolieren, Trocknen und Sieben des Komplexes umfassend.

11. Verfahren nach Anspruch 10, wobei der Komplex durch Zentrifugieren und Filtrieren isoliert wird.

12. Verfahren nach Anspruch 10, wobei der isolierte Komplex bei 40°-45°C getrocknet wird.

13. Verfahren nach Anspruch 10, ferner das Charakterisieren des Komplexes umfassend.

14. Verfahren Anspruch 13, wobei der Komplex durch Analysieren wenigstens einer der Eigenschaften des Komplexes charakterisiert wird.

15. Verfahren nach Anspruch 13, wobei der Komplex durch Festlegen seines pH-Löslichkeitsprofils, Festlegen seines Schmelzpunkts, Festlegen seines Wassergehalts, Analyse desselben durch Hochleistungs-Flüssigkeitschromatographie (HPLC) und/oder Analyse desselben durch Differenzialraster-Kalorimetrie (DSC) charakterisiert wird.

16. Verfahren nach Anspruch 8, wobei das basische Arzneimittel Fexofenadin ist.

17. Verfahren nach Anspruch 8, wobei das Säuresalz des basischen Arzneimittels ein Chlorhydratsalz von Fexofenadin ist.

18. Verfahren nach Anspruch 8, wobei der Komplex ein Fexofenadin-Carbomer-Komplex ist.

19. Verfahren nach Anspruch 8, wobei die eingesetzte Lauge ein Hydroxid eines Alkalimetalls ist.

20. Verfahren nach Anspruch 8, wobei die Lauge NaOH oder KOH ist.

21. Verfahren nach Anspruch 8, wobei die Lauge und das Salz des basischen Arzneimittels in äquimolaren Mengen bereitgestellt werden.

22. Verfahren nach Anspruch 8, wobei das saure Polymer ein Carbomer ist.

23. Verfahren nach Anspruch 8, wobei das Verhältnis des basischen Arzneimittels zu dem sauren Polymer in einem Bereich von 1:0,25 bis 1:1 liegt.

24. Verfahren nach Anspruch 8, wobei das Verhältnis des basischen Arzneimittels zu dem sauren Polymer in einem Bereich von 1:0,5 bis 1:0,6 liegt.

25. Verfahren nach Anspruch 8, ferner den Erhalt des Komplexes in einer geschmacksneutralen, granulären, direkt pressbaren, schnell löslichen und stabilen Form umfassend.

26. Verfahren nach Anspruch 8, umfassend
Entfernen eines Säuresalzes von Fexofenadin unter Verwenden einer Lauge in einem wässrigen Medium, wobei die Lauge und das Säuresalz von Fexofenadin in äquimolaren Mengen bereitgestellt werden; und
Umsetzen des Fexofenadins mit einem Carbomer in einem wässrigen Medium unter Bedingungen, die ausreichen einen Fexofenadin-Carbomer-Komplex zu bilden.

27. Pharmazeutische Formulierung, einen Fexofenadin-Carbomer-Komplex und einen pharmazeutisch verträglichen Arzneistoffträger umfassend.

28. Pharmazeutische Formulierung nach Anspruch 27, ferner wenigstens ein Mittel umfassend, ausgewählt aus Süßstoffen und Aromastoffen.

29. Pharmazeutische Formulierung nach Anspruch 27, ferner einen Süßstoff, ausgewählt aus der Gruppe, bestehend aus Sacchariden, Aspartam, Neotam und Steviosid, umfassend.

30. Pharmazeutische Formulierung nach Anspruch 27, ferner einen Aromastoff umfassend.

31. Pharmazeutische Formulierung nach Anspruch 27, ferner wenigstens ein Zerfallsmittel oder einen Zerfalls-/Lösungsverstärker umfassend.

32. Pharmazeutische Formulierung nach Anspruch 27, ferner wenigstens einen Zerfallsstoff, ausgewählt aus der Gruppe, bestehend aus Stärkederivaten, Cellulosen und Cellulosederivaten, umfassend.

33. Pharmazeutische Formulierung nach Anspruch 27, ferner einen Lösungs-/Zerfallsverstärker umfassend.

34. Pharmazeutische Formulierung nach Anspruch 27, feiner wenigstens einen Lösungs-/Zerfallsverstärker, ausgewählt aus der Gruppe, bestehend aus Zitronensäure, Natriumbicarbonat und Crosscarmellose-Natrium, umfassend.

35. Pharmazeutische Formulierung nach Anspruch 27, ferner einen Farbstoff umfassend.

36. Pharmazeutische Formulierung nach Anspruch 27, ferner ein Verdünnungsmittel, ausgewählt aus der Gruppe bestehend aus Mannitol, Stärken, Laktose, Saccharose, Sorbitol, Cellulosen und Mischung derselben, umfassend.

37. Pharmazeutische Formulierung nach Anspruch 27, ferner ein Schmiermittel umfassend.

38. Pharmazeutische Formulierung nach Anspruch 37, wobei das Schmiermittel ausgewählt ist aus der Gruppe, bestehend aus Talk, Magnesiumstearat, Calciumstearat, Polyethylenglykolen, kolloidem Siliciumdioxid, Fettsäuren, Glycerylestern von Fettsäuren und Mischungen derselben.

39. Pharmazeutische Formulierung nach Anspruch 27, wobei der Fexofenadin-Carbomer-Komplex in einer Menge in einem Bereich von 15 bis 40 Gewichtsprozent, basierend auf dem Gesamtgewicht der Formulierung, vorliegt.

40. Pharmazeutische Formulierung nach Anspruch 39, ferner ein Verdünnungsmittel umfassend, dass in einer Menge in einem Bereich von 30 bis 60 Gewichtsprozent, basierend auf dem Gesamtgewicht der Formulierung, vorliegt.

41. Pharmazeutische Formulierung nach Anspruch 27, wobei der Fexofenadin-Carbomer-Komplex geschmacksneutral, direkt pressbar und schnell löslich ist.

42. Pharmazeutische Formulierung nach Anspruch 27, wobei der Fexofenadin-Carbomer-Komplex erhalten wird durch:
in Kontakt bringen eines Fexofenadin-Säuresalzes mit einer Lauge; und
Umsetzen des Fexofenadins mit einem Carbomer unter Bedingungen, die ausreichend sind eine Komplexierung zu ermöglichen.

43. Pharmazeutische Formulierung nach Anspruch 42, wobei die Schritte des in Kontakt bringens und des Umsetzens in einem wässrigen Medium durchgeführt werden.

44. Pharmazeutische Formulierung nach Anspruch 27, wobei die Formulierung in einer festen oralen Dosierungsform besteht.

45. Pharmazeutische Formulierung nach Anspruch 44, wobei die feste orale Dosierungsform ausgewählt ist aus der Gruppe, bestehend aus Tabletten, Kapseln, Dragees, Granulat und Trockensirupen.

46. Pharmazeutische Formulierung nach Anspruch 27, wobei die Formulierung eine Wirksamkeit umfasst, die Fexofenadin-Hydrochlorid in einem Bereich von 30 mg bis 180 mg entspricht.

47. Pharmazeutische Formulierung nach Anspruch 27, wobei die Formulierung eine Wirksamkeit umfasst, die 30 mg Fexofenadin-Hydrochlorid entspricht.

48. Pharmazeutische Formulierung nach Anspruch 27, wobei die Formulierung eine Wirksamkeit umfasst, die 60 mg Fexofenadin-Hydrochlorid entspricht.

49. Pharmazeutische Formulierung nach Anspruch 27, wobei die Formulierung eine Wirksamkeit umfasst, die 120 mg Fexofenadin-Hydrochlorid entspricht.

50. Pharmazeutische Formulierung nach Anspruch 27, wobei die Formulierung eine Wirksamkeit umfasst, die 180 mg Fexofenadin-Hydrochlorid entspricht.

51. Pharmazeutische Formulierung nach Anspruch 27, wobei die Formulierung in Form einer Tablette besteht, die kaubar ist, zerlegbar ist, im Mund zerfällt und/oder sich auflöst.

52. Pharmazeutische Formulierung nach Anspruch 27, ferner ein Verdünnungsmittel, einen Süßstoff, einen Aromastoff, einen Farbstoff, ein Zerfallsmittel, einen Zerfallsverstärker, einen Löslichkeitsverstärker und/oder ein Schmiermittel umfassend.

53. Verfahren zum Herstellen einer einen Fexofenadin-Carbomer-Komplex und einen pharmazeutisch verträglichen Arzneistoffträger umfassenden pharmazeutischen Formulierung, wobei das Verfahren das Sieben des Fexofenadin-Carbomer-Komplexes in getrockneter Form durch ein oder mehrere Siebgewebe, zum Erhalt des Fexofenadin-Carbomer-Komplexes in granulärer Form mit einer gewünschten Partikelgröße und das Mischen des gesiebten Fexofenadin-Carbomer-Komplexes mit dem Arzneistoffträger für eine vorherbestimmte Zeitdauer unter gesteuerten Temperatur- und Feuchtigkeitsbedingungen, zum Bilden einer oralen festen Dosierungsformulierung, umfasst.

54. Verfahren nach Anspruch 53, ferner das Sieben des Arzneistoffträgers umfassend, bevor der gesiebte Fexofenadin-Carbomer-Komplex mit dem Arzneistoffsträger gemischt wird.

55. Verfahren nach Anspruch 53, ferner das Pressen der Mischung des Fexofenadin-Carbomer-Komplexes und des Arzneistoffsträgers, zum Herstellen der oralen festen Dosierungsformulierung in Form von Tabletten, umfassend.

## Revendications

1. Complexe de fexofénadine-carbomère comprenant de la fexofénadine complexée à un carbomère.

2. Complexe de fexofénadine-carbomère selon la revendication 1, dans lequel le rapport de fexofénadine au carbomère dans le complexe est compris dans la plage allant de 1/0,25 à 1/1.

3. Complexe de fexofénadine-carbomère selon la revendication 1, dans lequel le rapport de fexofénadine au carbomère dans le complexe est compris dans la plage allant de 1/0,5 à 1/0,6.

4. Complexe de fexofénadine-carbomère selon la revendication 1, dans lequel le complexe est insipide.

5. Complexe de fexofénadine-carbomère selon la revendication 4, dans lequel le complexe est directement compressible et à dissolution rapide.

6. Complexe de fexofénadine-carbomère selon la revendication 1, dans lequel le complexe est obtenu par :
la mise en contact d'un sel acide de fexofénadine avec un composé alcalin ; et
la réaction de la fexofénadine avec un carbomère dans des conditions efficaces pour permettre la complexation.

7. Complexe de fexofénadine-carbomère selon la revendication 6, dans lequel les étapes de mise en contact et de réaction sont réalisées dans un milieu aqueux.

8. Procédé de production d'un complexe d'un médicament de base comprenant les étapes de :
éliminer un sel acide du médicament de base en utilisant un composé alcalin ; et
faire réagir le médicament de base avec un polymère acide dans des conditions suffisantes pour former un complexe du médicament de base avec le polymère acide.

9. Procédé selon la revendication 8, dans lequel les étapes d'élimination et de réaction sont réalisées dans un milieu aqueux.

10. Procédé selon la revendication 8, comprenant en outre l'isolement, le séchage et le tamisage du complexe.

11. Procédé selon la revendication 10, dans lequel le complexe est isolé par centrifugation et filtration.

12. Procédé selon la revendication 10, dans lequel le complexe isolé est séché à 40-45 °C.

13. Procédé selon la revendication 10, comprenant en outre la caractérisation du complexe.

14. Procédé selon la revendication 13, dans lequel le complexe est **caractérisé par** l'analyse d'au moins une des propriétés du complexe.

15. Procédé selon la revendication 13, dans lequel le complexe est **caractérisé par** l'évaluation de son profile pH-solubilité, l'évaluation de son point de fusion, l'évaluation de sa teneur en eau, son analyse par chromatographie liquide haute performance (CLHP) et/ou par son analyse par colorimétrie différentielle à compensation de puissance (DSC).

16. Procédé selon la revendication 8, dans lequel le médicament de base est la fexofénadine.

17. Procédé selon la revendication 8, dans lequel le sel acide du médicament de base est un sel de chlorhydrate de fexofénadine.

18. Procédé selon la revendication 8, dans lequel le complexe est un complexe de fexofénad-ine-carbomère.

19. Procédé selon la revendication 8, dans lequel le composé alcalin utilisé est un hydroxyde d'un métal alcalin.

20. Procédé selon la revendication 8, dans lequel le composé alcalin est NaOH ou KOH.

21. Procédé selon la revendication 8, dans lequel le composé alcalin et le sel du médicament de base sont fournis dans des quantités équimolaires.

22. Procédé selon la revendication 8, dans lequel le polymère acide est un carbomère.

23. Procédé selon la revendication 8, dans lequel le rapport du médicament de base au polymère acide est compris dans la plage allant de 1/0,25 à 1/1.

24. Procédé selon la revendication 8, dans lequel le rapport du médicament de base au polymère acide est compris dans la plage allant de 1/0,5 à 1/0,6.

25. Procédé selon la revendication 8, comprenant en outre l'obtention du complexe sous une formé insipide, granulaire, directement compressible, à dissolution rapide et stable.

26. Procédé selon la revendication 8, comprenant les étapes de
élimine un sel acide de fexofénadine en utilisant un composé alcalin dans un milieu aqueux, où le composé alcalin et le sel acide de fexofénadine sont fournis en des quantités équimolaires ; et
faire réagir la fexofénadine avec un carbomère dans un milieu aqueux dans des conditions suffisantes pour former un complexe fexofénadine-carbomère.

27. Formulation pharmaceutique comprenant un complexe de fexofénadine-carbomère et un excipient pharmaceutiquement acceptable.

28. Formulation pharmaceutique selon la revendication 27, comprenant en outre au moins un agent sélectionné parmi les agents édulcorants et les agents aromatisants.

29. Formulation pharmaceutique selon la revendication 27, comprenant en outre un agent édulcorant sélectionné dans le groupe constitué des saccharides, de l'aspartame, du néotame et du stévioside.

30. Formulation pharmaceutique selon la revendication 27, comprenant en outre un agent aromatisant.

31. Formulation pharmaceutique selon la revendication 27, comprenant en outre au moins un agent de désagrégation et un améliorateur de désagrégation/dissolution.

32. Formulation pharmaceutique selon la revendication 27, comprenant en outre au moins un agent de désagrégation sélectionné dans le groupe constitué des dérivés d'amidon, de la cellulose et des dérivés de cellulose.

33. Formulation pharmaceutique selon la revendication 27, comprenant en outre un améliorateur de désagrégation/dissolution.

34. Formulation pharmaceutique selon la revendication 27, comprenant en outre au moins un améliorateur de désagrégation/dissolution sélectionné dans le groupe constitué de l'acide citrique, du bicarbonate de sodium et de la croscarmellose sodique.

35. Formulation pharmaceutique selon la revendication 27, comprenant en outre un agent de coloration.

36. Formulation pharmaceutique selon la revendication 27, comprenant en outre un diluant sélectionné dans le groupe constitué du mannitol, des amidons, du lactose, du saccharose, du sorbitol, des celluloses et de mélanges de ceux-ci.

37. Formulation pharmaceutique selon la revendication 27, comprenant en outre un lubrifiant.

38. Formulation pharmaceutique selon la revendication 37, dans lequel le lubrifiant est sélectionné dans le groupe constitué du talc, du stéarate de magnésium, du stéarate de calcium, des polyéthylène glycols, du dioxyde de silicium colloïdal, des acides gras, des esters glycéryliques d'acides gras et de mélanges de ceux-ci.

39. Formulation pharmaceutique selon la revendication 27, dans laquelle le complexe de fexofénadine-carbomère est présent en une quantité comprise dans la plage allant de 15 à 40 % en poids par rapport au poids total de la formulation.

40. Formulation pharmaceutique selon la revendication 39, comprenant en outre un diluant qui est présent en une quantité comprise dans la plage allant de 30 à 60 % en poids par rapport au poids total de la formulation.

41. Formulation pharmaceutique selon la revendication 27, dans laquelle le complexe de fexofénadine-carbomère est insipide, directement compressible et à dissolution rapide.

42. Formulation pharmaceutique selon la revendication 27, dans laquelle le complexe de fexofénadine-carbomère est obtenu par :
la mise en contact d'un sel acide de fexofénadine avec un composé alcalin ; et
la réaction de la fexofénadine avec un carbomère dans des conditions suffisantes pour permettre la complexation.

43. Formulation pharmaceutique selon la revendication 42, dans laquelle les étapes de mise en contact et de réaction sont réalisées dans un milieu aqueux.

44. Formulation pharmaceutique selon la revendication 27, dans laquelle la formulation est sous une forme posologique orale solide.

45. Formulation pharmaceutique selon la revendication 44, dans laquelle la forme posologique orale solide est sélectionnée dans le groupe constitué des comprimés, des gélules, des pilules, des granules et des sirops secs.

46. Formulation pharmaceutique selon la revendication 27, dans laquelle la formulation comprend un équivalent efficace de chlorhydrate de fexofénadine compris dans la plage allant de 30 mg à 180 mg.

47. Formulation pharmaceutique selon la revendication 27, dans laquelle la formulation comprend un équivalent efficace de 30 mg de chlorhydrate de fexofénadine.

48. Formulation pharmaceutique selon la revendication 27, dans laquelle la formulation comprend un équivalent efficace de 60 mg de chlorhydrate de fexofénadine.

49. Formulation pharmaceutique selon la revendication 27, dans laquelle la formulation comprend un équivalent efficace de 120 mg de chlorhydrate de fexofénadine.

50. Formulation pharmaceutique selon la revendication 27, dans laquelle la formulation comprend un équivalent efficace de 180 mg de chlorhydrate de fexofénadine.

51. Formulation pharmaceutique selon la revendication 27, dans laquelle la formulation est sous la forme d'un comprimé qui peut être croquer, est dispersible, se désagrège oralement et/ou se dissous dans la bouche.

52. Formulation pharmaceutique selon la revendication 27, comprenant en outre un diluant, un agent édulcorant, un agent aromatisant, un agent colorant, un agent de désagrégation, un améliorateur de désagrégation, un améliorateur de dissolution et/ou un lubrifiant.

53. Procédé de production de la formulation pharmaceutique comprenant un complexe de fexofénadine-carbomère et un excipient pharmaceutiquement acceptable, le procédé comprenant le tamisage du complexe de fexofénadine-carbomère sous une forme anhydre sur un ou plusieurs tamis pour obtenir le complexe de fexofénadine-carbomère sous une forme granulaire d'une taille de particule souhaitée et le mélangeage du complexe de fexofénadine-carbomère tamisé avec l'excipient pendant une durée de temps prédéterminée à une température contrôlée et dans des conditions humides pour former une formulation posologique solide orale.

54. Procédé selon la revendication 53, comprenant en outre le tamisage de l'excipient avant de mélanger le complexe de fexofénadine-carbomère avec l'excipient.

55. Procédé selon la revendication 53, comprenant en outre la compression du mélange du complexe de fexofénadine-carbomère et de l'excipient pour produire la formulation posologique solide orale sous forme de comprimés.
